# EUROPÄISCHE PATENTANMELDUNG

(11) **EP 1 867 472 A2**
(43) Veröffentlichungstag der Anmeldung: **19.12.2007**
(21) Anmeldenummer: 07010854.3
(22) Anmeldetag: 01.06.2007
(51) Int. Cl.: B32B 9/00, A61K 9/70, B32B 5/16, B32B 18/00, G03C 1/00, B05D 1/00, C01B 31/00

(54) **Verbundmaterial und Verfahren zum Herstellen eines derartigen Verbundmaterials**

(30) Priorität: 02.06.2006 DE 102006026302; 11.09.2006 DE 102006043216
(71) Anmelder: InovisCoat GmbH, 51368 Leverkusen (DE)
(72) Erfinder: Schmuck, Arno, Dr. rer. nat., 42799 Leichlingen (DE); Siegel, Jörg, Dr. rer. nat. habil., 51061 Köln (DE); Christian, Schönfeld Priv.-Doz. Dr. rer. nat. habil., 52511 Geilenkirchen (DE)
(74) Vertreter: Castell, Klaus

(57) **Zusammenfassung**

Um Verbundmaterialien weiterzuentwickeln, schlägt die Erfindung ein Verbundmaterial aus mindestens drei Schichten vor, bei welchem wenigstens eine der Schichten Wirkstoffe, keramische Nanopartilcel, Silbersalze oder nanopartikuläre Kohlenstoffmodifikationen aufweist.

## Beschreibung

Die Erfindung betrifft einerseits ein Verbundmaterial aus mindestens drei Schichten und andererseits ein Verfahren zum Herstellen eines Verbundmaterials mit wenigstens drei Schichten, bei welchen die Schichten auf einem Trägermaterial gegossen werden.

Verbundmaterialen sind aus dem Stand der Technik hinlänglich bekannt. Mittels derartiger Verbundmaterialien können besonders gute Eigenschaften, welchen Einzelmaterialien innewohnen, an einem einzigen Bauteil, eben einem Bauteil aus einem Verbundmaterial, miteinander verknüpft und genutzt werden.

Es ist Aufgabe vorliegender Erfindung Verbundmaterialien bereitzustellen, mit welchen neue Anwendungsbereiche erschlossen werden können.

Die Aufgabe der Erfindung wird von einem Verbundmaterial aus mindestens drei Schichten gelöst, wobei wenigstens eine der Schichten Wirkstoffe, keramische Nanopartikel, Silbersalze oder nanopartikuläre Kohlenstoffmodifikationen aufweist.

Vorliegend umschreibt der Begriff "Schichten" einzelne Materialgebiete des Verbundmaterials, die als Lagen übereinander geschichtet ein komplexes Verbundmaterial bilden.

Es versteht sich, dass im Sinne der vorliegenden Patentanmeldung das Verbundmaterial nahezu beliebig viele derartiger Schichten aufweisen kann. Vorliegend sind für das erfindungsgemäße Verbundmaterial jedoch in der Regel mindestens drei Schichten erforderlich, sodass wenigstens in einem Bereich eine der Schichten zumindest an zwei ihrer Hauptseiten vollständig von den weiteren Schichten bedeckt ist. Die Erfindung betrifft jedoch auch Verbundmaterialien mit weniger Schichten, sofern die Verbundmaterialien die genannten Wirkstoffe, Nanopartikel, Silbersalze oder Kohlenstoffmodifikationen aufweisen.

Die Aufgabe der Erfindung wird auch von einem Verbundmaterial aus mindestens drei Schichten gelöst, wobei das Verbundmaterial mittels eines Kaskadengießers oder eines Vorhanggießers hergestellt ist. Das Kaskadengießen bzw. das Vorhanggießen erlaubt vorteilhafter Weise das Aufbringen von mehreren Schichten, auch unterschiedlicher Dicke, auf ein Trägermaterial in einem Arbeitsgang. Somit ist das vorliegende Verbundmaterial mit einem geringen baulichen Aufwand herstellbar.

Verfahrenstechnisch wird die Erfindung von einem Verfahren zum Herstellen eines Verbundmaterials mit wenigstens drei Schichten, bei welchen die Schichten auf einem Trägermaterial gegossen werden, gelöst, welches sich dadurch auszeichnet, dass an eine mit einem ersten Bestandteil versetzte Schicht wenigstens eine mit einem von den ersten Bestandteil verschiedenen Bestandteil versetzte weitere Schicht gegossen wird.

Mittels des Aneinandergießens der unterschiedlichen Schichten lassen sich Verbundmaterialien herstellen, deren Schichten verschiedene Funktionalitäten aufweisen.

Das der Erfindung zugrunde liegende Verbundmaterial hat gegenüber bekannten Verbundmaterialien den weiteren Vorteil, dass vorliegend Schichten mit einer wesentlich höheren Präzision hinsichtlich der Schichtdicke erzielt werden können. Im Stand der Technik haben die Schichtdicken eine Toleranz von +/- 10 %. Bei dem erfindungsgemäßen Verbundmaterial weisen die einzelnen Schichtdicken eine Toleranz unterhalb +/-10 % auf. Es sind sogar Toleranzen von +/- 1 % bei den Schichtdicken der einzelnen Verbundmaterialschichten erzielbar.

Eine bevorzugte erste Ausführungsvariante des vorliegenden Verbundmaterials sieht vor, dass wenigstens eine der Schichten medizinische Wirkstoffe oder Bitterstoffe aufweist.

Es ist aus dem Stand der Technik bekannt, eine einschichtige Folie, welche beispielsweise wasserlöslich ist, mit medizinischen Wirkstoffen zu versehen. Mit dem Auflösen der einschichtigen Folie werden nach und nach die medizinischen Wirkstoffe freigegeben. Viele medizinische Wirkstoffe haben für einen Anwender, der einen medizinischen Wirkstoff oral einnehmen muss, jedoch einen unangenehmen Geschmack, sodass eine orale Verabreichung medizinischer Wirkstoffe meist problematisch ist.

Insbesondere bei Tieren ist eine Verabreichung medizinischer Wirkstoffe häufig erforderlich, da Haus- und Nutztiere beispielsweise regelmäßig entwurmt werden müssen. Vorwiegend gegen Bandwürmer wirkende Wirkstoffe werden von Katzen und Hunden nur sehr ungern oral aufgenommen. Insbesondere der Wirkstoff Praziquantel ist ein besonders bitter schmeckender Wirkstoff, der von Tieren freiwillig oral nicht aufgenommen wird. Die Einbringung der Wirkstoffe in Pasten oder Tabletten verbessert zwar die Aufnahmebereitschaft, führt allerdings vor allem bei Katzen noch nicht zu befriedigenden Ergebnissen.

Es wurde überraschend gefunden, dass mit vorliegendem Verbundmaterial beispielsweise ein übel schmeckender Wirkstoff in eine extrem dünne Schicht, auch in hoher Konzentration, eingebettet werden kann, wobei die dünne Schicht mit dem übel schmeckenden Wirkstoff von weiteren Schichten abgedeckt und eingekapselt ist. Die weiteren Schichten können vorliegend vorteilhafter Weise einen angenehm riechenden beziehungsweise angenehm schmeckenden Wirkstoff aufweisen, sodass die dünne Schicht mit dem übel schmeckenden Wirkstoff in dem Verbundmaterial besonders vorteilhaft eingebettet ist.

Zukünftig wird es zunehmend orale Applikationen zur Selbstmedikation geben. Viele medizinische Wirkstoffe in Medikamenten sind vom Geschmack oder Geruch jedoch unangenehm. Folglich ist es besonders vorteilhaft, wenn die die Wirkstoffe aufweisende Schicht eine mittlere Schicht im Verbundmaterial ist, welche von wenigstens zwei weiteren Schichten umgeben ist.

Insbesondere, wenn das vorliegende Verbundmaterial mit einer einen medizinischen Wirkstoff enthaltenden ersten Schicht erfindungsgemäß mittels eines Kaskadenverfahrens hergestellt ist, ist das Einkapseln der ersten Schicht sehr einfach realisiert.

Unangenehm wahrnehmbare Wirkstoffe im Verbundmaterial können zusätzlich ausgeblendet werden, wenn wenigstens eine der Schichten Geschmacksstoffe aufweist. Vorteilhafter Weise sind derartige Geschmacksstoffe in Schichten vorgesehen, welche die die Wirkstoffe enthaltende Schicht umkapseln.

Um eine Applikation von Wirkstoffen, welche in dem vorliegenden Verbundmaterial enthalten sind, an Lebewesen zu erleichtern, ist es vorteilhaft, wenn das Verbundmaterial einen zylindrischen Körper aufweist. Hierbei ist die Wirkstoffe enthaltende Schicht regelrecht eingewickelt, sodass das Verbundmaterial selbst gekaut werden kann, ohne dabei die Wirkstoffe enthaltende Schicht direkt zu beschädigen.

Ein weiteres bevorzugtes Ausführungsbeispiel sieht vor, das wenigstens eine der Schichten keramische Nanopartikel aufweist. Mit einem derartigen Verbundmaterial lassen sich auch dünne Keramikfolien herstellen. Insbesondere, wenn das zumindest dreischichtige Verbundmaterial mit einer keramische Nanopartikel aufweisenden Schicht mittels eines Kaskadengießverfahrens bzw. eines Vorhanggießverfahrens hergestellt ist, lassen sich deutlich dünnere Keramikfolien herstellen als aus dem Stand der Technik bekannt. Derartige Folien werden meist bisher mittels eines Rakelherstellungsverfahrens produziert. Dies ist jedoch aufwändig und es lassen sich damit weniger dünne Folien herstellen.

Insbesondere zeichnen sich die vorliegend erfindungsgemäß hergestellten Folien durch eine besonders hohe Gleichmäßigkeit der Schichten aus. Somit sind Folien extrem hoher Güte schnell und kostengünstig herstellbar.

Somit ist es im Zusammenhang mit dem der Erfindung zu Grunde liegenden Verbundmaterial vorteilhaft, wenn das Verbundmaterial eine Folie, insbesondere eine keramische Folie, umfasst.

Eine weitere vorteilhafte Ausführungsvariante sieht vor, dass wenigstens eine der Schichten sensibilisierte Silbersalze aufweist. In einer diesbezüglich besonders vorteilhaften Ausgestaltung sind die sensibilisierten Silbersalze mittels einer chemischen Reduktion elektrisch leitfähig gemacht. Somit sind mit dem vorliegenden Verbundmaterial besonders vorteilhaft elektrische Leiter realisiert.

Auch sieht eine vorteilhafte Ausführungsvariante vor, dass wenigstens eine der Schichten Carbon-Fullerene aufweist. Beispielsweise lassen sich mit dem erfindungsgemäßen dreischichtigen Verbundmaterial vorteilhaft fotoleitende Filme herstellen. Ein technischer Anwendungsbereich für derartige Filme liegt insbesondere bei Fotokopiergeräten.

Vorteilhafter Weise sind vorliegend Fullerene, insbesondere Carbon-Fullerene, in einem Polymer eingebettet.

Allen vorgenannten Verbundmaterialvarianten aus mindestens drei Schichten ist vorteilhafter Weise gemein, dass wenigstens eine der Schichten erste Bestandteile aufweist, welche von weiteren Bestandteilen weiterer Schichten verschieden sind.

Somit sieht eine Ausführungsvariante vor, dass die Schicht, welche wenigstens einen Bestandteil, nämlich Wirkstoffe, insbesondere medizinische Wirkstoffe oder Bitterstoff, keramische Nanopartikel, Silbersalze, insbesondere sensibilisierte Silbersalze, oder nanopartikuläre Kohlenstoffmodifikationen, insbesondere Carbon-Fullerene, aufweist, von wenigstens zwei weiteren Schichten umgeben ist.

Um eine erste Schicht des Verbundmaterials beidseitig mit weiteren Schichten abdecken und so vor der Umgebung abkapseln zu können, ist es vorteilhaft, wenn wenigstens zwei Schichten identische Eigenschaften aufweisen.

Darüber hinaus ist es vorteilhaft, wenn wenigstens zwei Schichten identische Bestandteile aufweisen. Insbesondere, wenn die eine erste Schicht mit ersten Bestandteilen beidseitig jeweils mit einer weiteren Schicht mit jeweils identischen Bestandteilen umgeben ist, ist ein symmetrisches Verbundmaterial realisiert.

Es versteht sich, dass die Schichten des vorliegenden Verbundmaterials relativ hohe Dicken aufweisen können. Insbesondere können die mit einem bekannten Kaskaden- bzw. Vorhanggießverfahren erzielbaren Schichtdicken realisiert werden. Um das Verbundmaterial jedoch möglichst kompakt bauen zu können, ist es vorteilhaft, wenn wenigstens eine der Schichten eine Schichtdicke von weniger als 20 µm oder von weniger als 0,5 µm aufweist. Hierdurch lassen sich auch besonders dünne Folien mit dem vorliegenden Verbundmaterial realisieren.

Es wurde gefunden, dass selbst in extrem dünnen Verbundmaterialschichten eine genügend große Menge an Bestandteilen, wie medizinischen Wirkstoffen, eingebunden werden können. Um in einzelne Schichten des Verbundmaterials auch größere Mengen an Bestandteilen einarbeiten zu können, ist es vorteilhaft, wenn wenigstens eine der Schichten eine Schichtdicke von mehr als 0,1 µm oder von mehr als 0,4 µm aufweist.

Bestandteile einer der Schichten können baulich besonders einfach in eine der Schichten impliziert werden, wenn wenigstens eine der Schichten Gelatine aufweist.

Eine weitere Verbundmaterialvariante sieht vor, dass wenigstens eine der Schichten eine keramische Suspension mit weniger als 80 Gewichts-%, vorzugsweise weniger als 70 Gewichts-%, an keramischen Nanopartikeln aufweist.

Darüber hinaus ist es vorteilhaft, wenn wenigstens eine der Schichten eine keramische Suspension mit mehr als 30 Gewichts-%, vorzugsweise mehr als 45 Gewichts-%, an keramischen Nanopartikeln aufweist.

Eine Menge an keramischen Nanopartikeln in den vorgenannten Grenzen ist günstig für eine vorteilhafte Realisierung von keramischen Folien.

Wenn eine Schicht des vorliegenden Verbundmaterials keramische Nanopartikel aufweist, ist es vorteilhaft, wenn die keramischen Nanopartikel eine Korngröße von weniger als 5000 nm, vorzugsweise von weniger als 1000 nm, aufweisen.

Darüber hinaus ist es vorteilhaft, wenn die keramischen Nanopartikel eine Korngröße von mehr als 10 nm, vorzugsweise von mehr als 100 nm, aufweisen.

Es sind jedoch Anwendungsgebiete, wie etwa im Zusammenhang mit Brennstoffzellen, bekannt, bei welchen Folien mit extrem kleine Porengrößen realisiert werden müssen. Hier hat es sich gezeigt, dass Nanopartikel mit Korngrößen zwischen 1 nm und 100 nm zum Einsatz gelangen müssen, so dass vorliegend auch derartige Korngrößen vorteilhaft sein können.

Um Bestandteile, welche in einer Schicht enthalten sind, in dieser Schicht zu stabilisieren, ist es vorteilhaft, wenn wenigstens eine der Schichten einen Verdicker, ein Härtungsmittel für einen Bestandteil einer Schicht, wie etwa Gelatine, oder ein Vernetzungsmittel für einen Bestandteil einer Schicht, wie etwa Gelatine, aufweist.

Es ist vorteilhaft, wenn eine der Schichten eine elektrisch funktionalisierte Folie ist. Mittels des erfindungsgemäßen Verbundmaterials lassen sich vorteilhafter Weise baulich besonders einfach sehr dünne elektrische Leiter bereitstellen.

Insbesondere, wenn das Verbundmaterial elektrisch leitend ist, also einen elektrischen Leiter bilden beziehungsweise bereit stellen soll, ist es vorteilhaft, wenn wenigstens eine der Schichten elektrisch leitfähig ist.

Ein besonders hochwertiges elektrisch leitfähiges Verbundmaterial liegt vor, wenn wenigstens eine elektrisch leitfähige Schicht Silber und/oder Silbersalz aufweist. Als Silbersalz können vorliegend bevorzugt Silberchlorid, Silberbromid, Silberjodid oder Mischformen davon verwendet werden.

Ein elektrisch isolierter Leiter in Gestalt des vorliegenden Verbundmaterials ist bereit gestellt, wenn eine elektrisch leitfähige Schicht von elektrisch isolierenden Schichten umgeben ist.

Es ist weiter vorteilhaft, wenn die elektrisch isolierenden Schichten ein Polymer, wie Gelatine, als Isolator aufweisen. Es versteht sich, dass neben einem Polymer auch jegliche andere Materialien als Isolator eingesetzt werden können, solange diese dazu geeignet sind, eine isolierende Schicht des vorliegenden Verbundmaterials zu bilden.

Sollte das Verbundmaterial eine oder mehrere Schichten mit Silbersalzen aufweisen, ist es vorteilhaft, wenn die Silbersalze in einem Bereich oberhalb von 300 nm, vorzugsweise oberhalb von 350 nm, sensibilisiert sind.

Darüber hinaus ist es vorteilhaft, wenn die Silbersalze in einem Bereich unterhalb von 800 nm, vorzugsweise unterhalb von 750 nm, sensibilisiert sind.

Die Vorteile der für unterschiedliche Wellenlängenbereiche beispielsweise sensibilisierten Silberhalogenidsalze für das vorliegende Verbundmaterial liegen darin, dass Vorteilhafter Weise unterschiedliche Leiterbahnstrukturen in verschiedenen Schichten in einem Schritt aufbelichtet werden können. Hierbei werden die Leiterbahngitter (grids) mit unterschiedlichen Sensibilisatoren für bestimmte Wellenlängenbereiche ausgerüstet und vorzugsweise mit Licht unterschiedlicher Wellenlänge belichtet. Beispielsweise wird ein Leiterbahngitter A in einer Schicht X mit Licht der Wellenlänge 380 nm bis 480 nm (blaues Licht) und ein Leiterbahngitter B in einer weiteren Schicht Y mit Licht der Wellenlänge 590 nm bis 800 nm (rotes Licht) belichtet. In einer daran anschließenden chemischen Verarbeitung entstehen gleichzeitig zwei unterscheidbare Leiterbahngitter (grids).

Eine diesbezügliche Ausführungsvariante sieht vor, dass die Silbersalze mit Ammoniumthiosulfat oder mit Natriumthiosulfat fixiert sind.

Um Verbundmaterialien herzustellen, die speziell auf bestimmte Applikationen abgestimmt sind, ist es vorteilhaft, wenn wenigstens zwei Schichten voneinander verschiedene Schichtdicken aufweisen.

Zum Herstellen des vorliegenden Verbundmaterials, aber auch zur weiteren Handhabung dieses aus mindestens drei Schichten bestehenden Verbundmaterials, ist es vorteilhaft, wenn wenigstens eine der Schichten an einem Trägermaterial angeordnet ist. Hierbei ist es vorliegend applikationsspezifisch, ob das Verbundmaterial an dem Trägermaterial angeordnet bleibt oder von diesem zur eigentlichen Verwendung abgezogen wird.

Damit bei der Herstellung des Verbundmaterials, insbesondere bei einem Brennvorgang von einzelnen Folien aus dem Verbundmaterial, das Verbundmaterial nicht oder nur wenig verformt wird, ist es vorteilhaft, wenn das Verbundmaterial symmetrisch ausgebildet ist.

Insbesondere, wenn es sich bei einem Verbundmaterial um eine lösliche Folie handelt, ist es vorteilhaft, wenn polymerhaltige Schichten ein wasserlösliches Polymer umfassen.

Weitere Vorteile, Ziele und Eigenschaften vorliegender Erfindung werden anhand nachfolgender Erläuterung anliegender Zeichnung beschrieben, in welcher beispielhaft unterschiedliche Verbundmaterialien und deren Anwendungsbereiche sowie deren Zusammensetzungen beschrieben sind.

Es zeigen
- Figur 1: schematisch einen Längsschnitt durch ein Verbundmaterial an einer Trägerschicht, wobei eine mit medizinischen Wirkstoffen versetzte erste Schicht von einer zweiten Schicht und einer dritten Schicht, welche Geschmacksstoffe aufweisen, umgeben ist,
- Figur 2: schematisch einen Längsschnitt durch ein weiteres Verbundmaterial an einer Trägerschicht, wobei eine mit keramischen Nanopartikeln versetzte erste Schicht von einer zweiten Schicht ohne derartige keramische Nanopartikel und einer dritten Schicht ohne derartige keramische Nanopartikel umgeben ist,
- Figur 3: schematisch einen Längsschnitt durch ein alternatives Verbundmaterial an einer Trägerschicht, wobei eine mit einem spektral sensibilisierten Silbersalz versetzte erste Schicht von einer zweiten Schicht und einer dritten Schicht aus einem nicht leitendem Polymer umgeben ist und
- Figur 4: schematisch einen Längsschnitt durch ein weiteres Verbundmaterial an einer Trägerschicht, wobei eine mit Nano-CarbonFullerenen versetzte erste Schicht von einer zweiten Schicht ohne derartige Nano-Carbon-Fullerene und einer dritten Schicht ohne derartige Nano-Carbon-Fullerene umgeben ist.

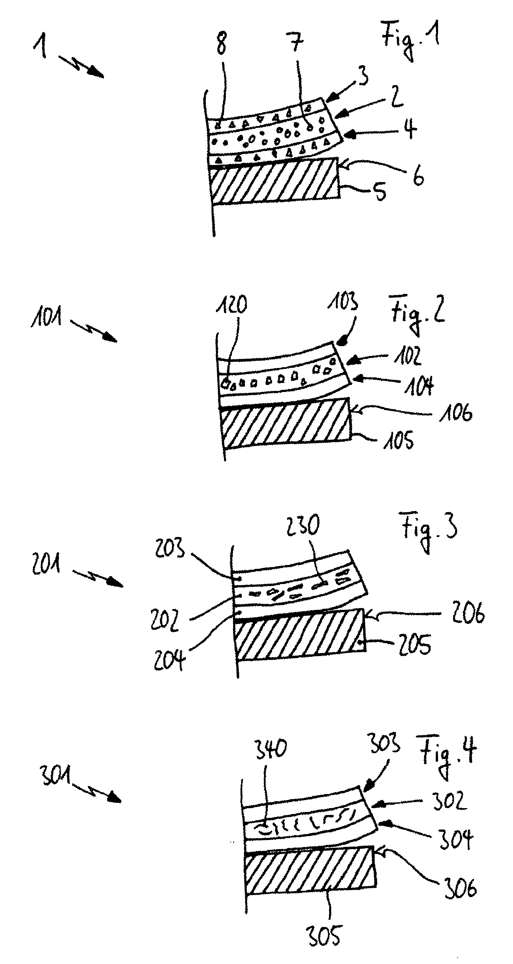

Das in der Figur 1 gezeigte Verbundmaterial 1 besteht aus einer mittleren Schicht 2, einer oberen Schicht 3 und einer unteren Schicht 4, welche an einem Trägermaterial 5 angeordnet ist. Im vorderen Bereich 6 des Verbundmaterials 1 ist die untere Schicht 4 bereits etwas von dem Trägermaterial 5 gelöst.

Die mittlere Schicht 2 umfasst in diesem Ausführungsbeispiel Bestandteile eines medizinischen Wirkstoffes 7 und ist von der oberen und der unteren Schicht 3 und 4 vollständig umgeben.

Sowohl die obere Schicht 3 als auch die untere Schicht 4 umfassen Geschmacksstoffe 8, welche in diesem Ausführungsbeispiel für eine Sinneswahrnehmung eines Tieres angenehm sind.

Alle drei Schichten 2, 3 und 4 des Verbundmaterials 1 sind miteinander verschmolzen. Die drei Schichten 2, 3 und 4 des vorliegenden Verbundmaterials 1 wurden mittels eines bekannten Kaskadengießverfahrens aneinander gefügt.

Auf Grund der Tatsache, dass die mittlere Schicht 2 mit ihrem medizinischen Wirkstoff 7 mittels der den Geschmacksstoffe 8 umfassenden oberen und unteren Schicht 3 und 4 vollständig eingekapselt ist, eignet sich das vorliegende Verbundmaterial 1 ausgezeichnet dazu, einen medizinischen Wirkstoff 7, insbesondere einem Tier, oral zu verabreichen.

Wie eingangs erwähnt, wird es zunehmend orale Applikationen zur Selbstmedikation geben. Viele Wirkstoffe in Medikamenten sind vom Geschmack oder Geruch unangenehm. Überraschend wurde gefunden, dass ein mittels eines Kaskadengießverfahrens hergestelltes Verbundmaterial 1 einen übel schmeckenden medizinischen Wirkstoff 7 in extrem dünnen Schichten 2 in hoher Konzentration enthalten und doch mit mehreren Schichten 3, 4 geschmacklich wirksam überdeckt werden kann.

Der Abbau der im Sinne der Patentanmeldung wohlschmeckenden Schichten 3, 4 kann durch die Wahl einer Matrix, die aus Naturstoffen wie Gelatine, Cellulose oder Chitinen aufgebaut sein kann, und die Wahl eines geeigneten Vernetzungsmittels (Härters) so gesteuert werden, dass selbst bei längerem Kauen oder Lutschen der medizinische Wirkstoff 7 nicht im Mund oder an den Geschmacksorganen freigesetzt wird.

Auf einen Träger 5 wird mittels eines Kaskadengießers ein aus mindestens drei Schichten 2, 3 und 4 bestehendes Gießpaket [A-**B**-A] aufgetragen, dessen erste und letzte Schicht 3 und 4 einen Geruchs-, bzw. Geschmacksstoff 8 enthält, der in unterschiedlichen Konzentrationen eingebracht werden kann. Dieser Geruchs- bzw. Geschmacksstoff 8 ist frei wählbar. Die mittlere Schicht 2 kann den medizinischen Wirkstoff 7 enthalten. Dieser Aufbau (drei Schichten) kann vom Trägermaterial 5 abgezogen (gestrippt) und konfektioniert werden.

Es können beispielsweise Pellets hergestellt werden, die eine definierte Wirkstoffmenge enthalten. Möglich wäre auch eine Dosierung der abgestrippten Folien über die zur Verfügung stehende Fläche einer der Schichten 2, 3 und/oder 4. Der medizinische Wirkstoff 7 ist beispielsweise in einen Gelatineverbund so einzubetten, dass durch geruchs- und geschmacksmaskierende Gelatineschichten eine freiwillige Aufnahme der medizinischen Wirkstoffe 7 beispielsweise durch ein Tier erfolgt sowie die Freisetzung der medizinischen Wirkstoffe 7 erst im Magen und nicht schon bei der Aufnahme im Mund stattfindet und so eine hohe freiwillige Aufnahme erfolgt.

Es versteht sich, dass das vorliegende Verbundmaterial 1 auch bei Menschen, insbesondere bei Kindern, vorteilhaft verwendet werden kann.

Die Herstellung einer Gießlösung für eine Schicht 2, 3 und 4 kann folgendermaßen vorgenommen werden. Die eingesetzten medizinischen Wirkstoffe 7 sind so gut wie wasserunlöslich und lassen sich auch nur in geringen Mengen in Alkohol lösen. Daher wurde eine Suspension in Gelatine gefertigt. Um eine gleichmäßige Verteilung der medizinischen Wirkstoffe 7 zu gewährleisten und gröbere Partikel zu zerschlagen (Praziquantel bildet z.B. lange Nadeln) bewährte sich folgende Vorgehensweise:

Gelatine wird in kaltem Wasser aufgequollen und anschließend bei 40 °C gelöst. Die Wirkstoffkombination wird in die Gelatinelösung gegeben und beispielsweise mit einem Ultra-Turrax-Rührer (Rührstab mit einem sehr schnell rotierenden Messer, bis zu 24.000 U/Min) drei Minuten bei 3000 U/Min gerührt. Durch die hohe Umdrehungszahl des Messers treten hohe Scherkräfte auf und es gelingt, Partikel in einer Suspension sehr klein zu zerhacken. Vorliegend sind folgende Einsatzstoffe erforderlich:

| | |
|---|---|
| I. Gelatine: | Pharmagelatine von Gelita |
| | 140 Bloom, |
| | 160 Bloom |
| | oder 200 Bloom |

Die Bloomzahl gibt die Gelierkraft einer Gelatinesorte an und ist somit auch ein Parameter für die Löslichkeit in Wasser.
II. Glycerin: Glycerin wird vorteilhafter Weise als Weichmacher in die Gießlösungen dazugegeben, da der Schichtverband ohne diesen Zusatz sehr spröde wird und dies eine Weiterverarbeitung erschweren würde.
III. Unterlage: Da die Schichten sich von der Unterlage abstrippen lassen müssen, wird eine unsubstrierte Polyesterunterlage gewählt.

Formulierung Wirkstoffkombination Katze (5kg) :

Kombination: Pyrantelembonat 59 mg/kg, γ = 295 mg/5 kg, Praziquantel 5 mg/kg, γ = 25 mg/5 kg, Summe: 320 mg Wirkstoff pro Dosiseinheit.

**Schichtaufbau:**

| | Schichtdicke nass |
|---|---|
| Gelatine | 180 µm |
| Wirkstoffkombination | 180 µm |
| Gelatine | 180 µm |

Gesamt -
Schichtdicke nach der Trocknung: 55 µm.

Rezept Gießlösungen:

**Außenschichten**

| | |
|---|---|
| 250 g | Gelatine |
| 2190 g | Wasser |
| 60 g | Glycerin |
| 2500 g | Gießlösung |

**Wirkstoffschicht:**

| | |
|---|---|
| 125 g | Gelatine |
| 1001 g | Wasser |
| 30 g | Glycerin |
| 86,43 g | Pyrantelembonat |
| 7,32 g | Praziquantel |
| 1250 g | Gießlösung |

Ein 230 cm² großes Folienstück enthält 320 mg der Wirkstoffkombination für eine 5 kg schwere Katze.

Formulierung Wirkstoffkombination Hund (15kg) :

Kombination: Pyrantelembonat 14,5 mg/kg, γ = 217,5 mg/5 kg, Praziquantel 5 mg/kg, γ = 75 mg/5 kg, Febantel 15 mg/kg, γ = 225 mg/5 kg, Summe: 517,5 mg Wirkstoff pro Dosiseinheit.

Zusätzlich wurde in die Außenschichten ein Flavor (Fleischgeschmack) eingearbeitet. Die Einarbeitung erfolgte wie oben beschrieben mit einem Ultra-Turrax-Rührer.

**Schichtaufbau:**

| | Schichtdicke nass |
|---|---|
| Gelatine + Flavor | 180 µm |
| Wirkstoffkombination | 180 µm |
| Gelatine + Flavor | 180 µm |

Gesamt-Schichtdicke nach der Trocknung : 55 µm.

**Rezept Gießlösungen :**

| | | |
|---|---|---|
| Außenschichten | 250 g | Gelatine |
| | 2178 g | Wasser |
| | 62,4 g | Glycerin |
| | 9,83 g | Flavor |
| | 2500 g | Gießlösung |

| | | |
|---|---|---|
| Wirkstoffschicht: | 125 g | Gelatine |
| | 1000 g | Wasser |
| | 31,2 g | Glycerin |
| | 39,4 g | Pyrantelembonat |
| | 13,5 g | Praziquantel |
| | 8 | |
| | 40,7 g | Febantel |
| | 6 | |
| | 1250 g | Gießlösung |

Ein 372,15 cm² großes Folienstück enthält 517,5 mg der Wirkstoffkombination für einen 15 kg schweren Hund. Darreichungsformen:

Aus der abgestrippten Folie werden Stücke ausgeschnitten (mit einer vorzugsweise genau definierten Wirkstoffmenge). Diese Wirkstoffmengen können geschräddert oder am Stück in Feuchtfutter eingearbeitet werden. Die Gelatine quillt zwar in feuchter Umgebung, hält aber die medizinischen Wirkstoffe 7 im festen Verbund. Aus der Folie werden längliche Stücke ausgeschnitten, die anschließend aufgerollt werden, so dass eine zylindrische Form entsteht. Folgende Zylinder wurden realisiert:

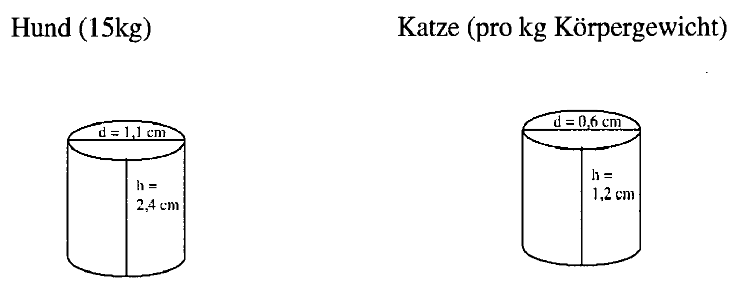

Im Vergleich mit bisherigen Darreichungsformen konnten für Hunde in durchgeführten Feldtests folgende vorteilhafte Ergebnisse erzielt werden:

| Darreichungsform | Paste | Tablette | Folie (weiterverarbeitet als Rolle) | Folie in Weichfutter verabreicht |
|---|---|---|---|---|
| % freiwillige Aufnahme | 60 | 68 | 95 | 98 |

Im Vergleich mit bisherigen Darreichungsformen konnten für Katzen in durchgeführten Feldtests folgende Ergebnisse erzielt werden:

| Darreichungsform | Paste | Tablette | Folie (weiterverarbeitet als Rolle) | Folie in Weichfutter verabreicht |
|---|---|---|---|---|
| % freiwillige Aufnahme | 55 | 54 | 90 | 94 |

Das in der Figur 2 gezeigte Verbundmaterial 101 besteht aus einer mittleren Schicht 102, einer oberen Schicht 103 und einer unteren Schicht 104, welche an einem Trägermaterial 105 angeordnet ist. Im vorderen Bereich 106 des Verbundmaterials 101 ist die untere Schicht 104 bereits etwas von dem Trägermaterial 105 gelöst.

Die mittlere Schicht 102 umfasst in diesem Ausführungsbeispiel keramische Nanopartikel 120 und ist von der oberen und der unteren Schicht 103 und 104, welche derartige keramische Nanopartikel 120 nicht umfassen, vollständig umgeben. Die obere und untere Schicht 103, 104 basieren auf einer Gelatine.

Alle drei Schichten 102, 103 und 104 des Verbundmaterials 101 sind miteinander verschmolzen. Die drei Schichten 102, 103 und 104 des vorliegenden Verbundmaterials 101 wurden mittels eines bekannten Kaskadengießverfahrens aneinander gefügt.

Mittels des Einsatzes von Kaskaden- oder Vorhanggießern lassen sich gegenüber dem Stand der Technik insbesondere deutlich dünnere Keramikfolien herstellen und/oder die Gleichmäßigkeit der Schichten 102, 103, 104 deutlich verbessern.

Dazu werden die keramischen Nanopartikel 120 vorzugsweise in eine auf Gelatine basierende Gießlösung eingebracht. Hierbei wurde überraschend auch gefunden, dass bis zu 10 mit keramischen Nanopartikeln versehene Schichten als unterschiedlich funktionalisierte Einzelschichten, die auch unterschiedliche keramische Partikel in unterschiedlichen Korngrößen und Konzentrationen enthalten können, in einem einzigen Arbeitsgang auf einen Träger aufgebracht werden können, wenn man hierzu vorzugsweise ein Kaskaden- oder Vorhanggießverfahren verwendet.

Die als Folien ausgebildeten Schichten 103 und 104 können symmetrisch oder asymmetrisch um eine funktionale mittlere Schicht 102 aufgebaut sein. Ein symmetrischer Aufbau [A-B-C-D-C-B-A] hat den Vorteil, dass sich derartige Folien beim Brennvorgang nicht oder nur sehr wenig verformen.

Ein weiterer Vorteil liegt in der Möglichkeit, vielschichtige keramische Folien fertigen zu können, die sich durch extrem dünne Schichtdicken auszeichnen. Die Herstellung der Keramikfolien (Schichten mit den keramischen Nanopartikeln) erfolgt, wie vorstehend beschrieben, vorzugsweise im Kaskadengießverfahren.

Als Unterlage (Trägerschicht 105) kann im Allgemeinen vorteilhafter Weise eine unsubstrierte Polyesterbahn gewählt werden, da die Folien von der Unterlage abgestrippt werden.

Als erste Schicht wird in diesem Ausführungsbeispiel vorteilhaft eine dünne Gelatineschicht gegossen, die das Abstrippen erleichtert.

**Gelatinerezeptur:**

| | |
|---|---|
| 100 g | Gelatine |
| 890 g | Wasser |
| 10 g | Glycerin |
| 1000 g | Gießlösung |

I. Folien mit Aluminiumoxid-Nanopartikel:

**Gießlösung 1:**

| | Menge (g) |
|---|---|
| Aluminiumoxid d₅₀ = 1µm | 3800 |
| Gelatine 5%ig | 1600 |
| Verflüssiger | 8 |
| Netzmittel | 75 |
| Glycerin | 30 |
| | |
| Summe | 5.438 |

**Gießlösung 2:**

| | Menge (g) |
|---|---|
| Aluminiumoxid d₅₀ = 2,5µm | 3800 |
| Gelatine 5%ig | 1600 |
| Verflüssiger | 8 |
| Netzmittel | 75 |
| Glycerin | 30 |
| | |
| Summe | 5.438 |

**Gießlösung 3:**

| | Menge (g) |
|---|---|
| Aluminiumoxid d₅₀ = 4 µm | 3800 |
| Gelatine 5%ig | 1600 |
| Verflüssiger | 8 |
| Netzmittel | 75 |
| Glycerin | 30 |
| | |
| Summe | 5.438 |
| | |

**Schichtaufbau 1:**

| | Schichtdicke nass |
|---|---|
| Gelatine (10%) | 30 µm |
| Gießlösung 1 | 60 µm |
| Gießlösung 2 | 60 µm |
| Gießlösung 3 | 60 µm |
| Trockenschichtdicke: 158 µm | |

**Schichtaufbau 2:**

| | Schichtdicke nass |
|---|---|
| Gelatine (10%) | 30 µm |
| Gießlösung 1 | 60 µm |
| Gießlösung 2 | 60 µm |
| Gelatine (10%) | 30 µm |
| Trockenschichtdicke: 92,04 µm | |

**Schichtaufbau 3:**

| | Schichtdicke nass |
|---|---|
| Gelatine (10%) | 30 µm |
| Gießlösung 1 | 60 µm |
| Trockenschichtdicke: 46,02 µm | |

**Schichtaufbau 3:**

| | Schichtdicke nass |
|---|---|
| Gelatine (10%) | 10 µm |
| Gießlösung 3 | 20 µm |
| Trockenschichtdicke: 15,34 µm | |

II. Folien aus Zirkoniumoxid :

**Gießlösung 1:**

| | Menge (g) |
|---|---|
| Zirkoniumoxid d₅₀ =1,3µm | 3150 |
| Gelatine 10%ig | 1850 |
| Verflüssiger | 6,5 |
| Netzmittel | 40 |
| Glycerin | 25 |
| | |
| Summe | 5071,5 |

**Gießlösung 2:**

| | Menge (g) |
|---|---|
| Zirkoniumoxid d₅₀ = 3,5µm | 3150 |
| Gelatine 5%ig | 1850 |
| Verflüssiger | 6,5 |
| Netzmittel | 40 |
| Glycerin | 25 |
| | |
| Summe | 5071,5 |

**Schichtaufbau 1:**

| | Schichtdicke nass |
|---|---|
| Gelatine (10%) | 20 µm |
| Gießlösung 1 | 20 µm |
| Gießlösung 2 | 40 µm |
| Trockenschichtdicke: 39,8 µm | |

**Schichtaufbau 2:**

| | Schichtdicke nass |
|---|---|
| Gelatine (10%) | 30 µm |
| Gießlösung 1 | 20 µm |
| Trockenschichtdicke: 15,6 µm | |

### III. Folien aus Siliciumcarbid:

**Gießlösung 1:**

| | Menge (g) |
|---|---|
| Siliciumcarbid d₅₀ = 0,8 µm | 2900 |
| Gelatine 5 %ig | 1700 |
| Verflüssiger | 8 |
| Netzmittel | 35 |
| Glycerin | 30 |
| | |
| Summe | 4673 |

**Schichtaufbau 1:**

| | Schichtdicke nass |
|---|---|
| Gelatine (10%) | 10 µm |
| Gießlösung 1 | 20 µm |
| Trockenschichtdicke: 13,8 µm | |

**Schichtaufbau 2:**

| | Schichtdicke nass |
|---|---|
| Gelatine (10%) | 10 µm |
| Gießlösung 1 | 10 µm |
| Trockenschichtdicke: 6,4 µm | |

Die erfindungsgemäße Technologie schafft in der Herstellung folgende tabellarisch zusammengefassten Vorteile:

| | Herstellungsdicke | Dickentoleranzen |
|---|---|---|
| Stand der Technik | > 30 µm | 50 µm +/- 5 µm |
| Erfindungsgemäße Technik | > 5 µm | 50 µm +/- 1 µm |

Das in der Figur 3 gezeigte Verbundmaterial 201 besteht aus einer mittleren Schicht 202, einer oberen Schicht 203 und einer unteren Schicht 204, welche an einem Trägermaterial 205 angeordnet ist. Im vorderen Bereich 206 des Verbundmaterials 201 ist die untere Schicht 204 bereits etwas von dem Trägermaterial 205 gelöst.

Die mittlere Schicht 202 ist in diesem Ausführungsbeispiel elektrisch leitend und von der oberen elektrisch nicht leitenden Schicht 203 und der unteren elektrisch nicht leitenden Schicht 204 vollständig umgeben. Die obere und untere Schicht 203, 204 basieren auf einer Gelatine. Die mittlere Schicht 202 enthält in diesem Ausführungsbeispiel ein spektral sensibilisiertes Silbersalz 230.

Alle drei Schichten 202, 203 und 204 des Verbundmaterials 201 sind miteinander verschmolzen. Die drei Schichten 202, 203 und 204 des vorliegenden Verbundmaterials 201 wurden mittels eines bekannten Kaskadengießverfahrens aneinander gefügt.

Mittels des vorliegenden Verbundmaterials 201 ist ein vorteilhafter elektrischer Leiter mit einem elektrisch nicht leitenden Polymer bereit gestellt. Dieses Polymer ist vorliegend Gelatine.

Überraschend wurde gefunden, dass in einem Schichtverband, bestehend aus drei Schichten 202, 203 und 204 [A-**B**-C], durch Belichten, Reduktion und Fixieren elektrische Leiter erzeugt werden können, die sich durch eine hohe Trennschärfe auszeichnen. Die Schichten A und C sind üblicherweise nicht elektrisch leitend und bestehen aus einem Polymer, beispielsweise Gelatine. Eine mittlere Schicht B enthält ein spektral sensibilisiertes Silbersalz, wie Silberchlorid, Silberbromid oder Silberjodid oder Mischformen davon. Durch eine Belichtung und anschließende photographisch analoge Reduktion, wie beispielsweise mit einem Hydrochinon oder Ascorbinsäure, kann elementares Silber abgeschieden werden. Das so abgeschiedene elementare Silber befindet sich noch in einer Silbersalzumgebung, die vorteilhafter Weise mittels einer Fixage mit Kalium- oder Natrium- oder Ammoniumthiosulfat aus dem Schichtverband (Verbundmaterial 201) herausgelöst wird. Beispielsweise von photographischen Materialien ist bekannt, dass Gelatine für Silbersalze ein Schutzkolloid darstellt, welches die hier verwendeten Silbersalze stabilisiert.

Durch eine für verschiedene Wellenlängenbereiche des Lichtes ausgelegte Sensibilisierung der Silberhalogenid-Nanopartikel ist es möglich, unterschiedlich definierte Leiterbahnen gleichzeitig zu erzeugen. Etwa in einer Schichtfolge [A-**B**-C-**D**-C-**E**-A] lassen sich beispielsweise in den Schichten B, D und E drei unterschiedliche Schaltkreise realisieren. Diese Silber enthaltenden Leiter zeichnen sich neben der größeren Variabilität in der mehrschichtigen Strukturierung durch eine bessere Leitfähigkeit gegenüber dem Stand der Technik aus.

Beispielsweise wird ein fotografisches Aufzeichnungsmaterial, welches für einen Schnellverarbeitungsprozess geeignet ist, hergestellt, indem auf ein beidseitig mit Polyethylen beschichtetes Papier die folgenden Schichten in der angegebenen Reihenfolge aufgetragen wurden.

Die nachstehenden Mengenangaben beziehen sich jeweils auf 1 m². Für den Silberhalogenidauftrag sind die entsprechenden Mengen AgNO₃ in g/m² angegeben.
I. Herstellung der Silberhalogenidemulsion:
   Lösung 1:
      6000 g demineralisiertes Wasser
      180 g Gelatine
      10g NaCl
      14 ml Schwefelsäure (25 gew.-%ig)
   Lösung 2:
      1400 g demineralisiertes Wasser
      57 g NaCl
      112gKBr
   Lösung 3:
      1400 g demineralisiertes Wasser
      320 g AgNO₃
   Lösung 4:
      1800 g demineralisiertes Wasser
      132 g NaCl
      238 g KBr
      0,4 mg K₂lrCl₆
      0,076 mg RhCl₃
   Lösung 5:
      1800 g demineralisiertes Wasser
      680 g AgNO₃

Die Lösung 1 wird vorgelegt und auf 65° C erwärmt. Unter Konstanthalten dieser Temperatur werden die Lösungen 2 und 3 gleichzeitig bei einem pAg-Wert von 8 der Lösung 1 innerhalb von 35 Minuten zugegeben. Dann werden die Lösungen 4 und 5 gleichzeitig unter Einhaltung von pAg 8 bei 65 °C in 45 Minuten zugegeben. Es wird eine Silberchloridbromidemulsion mit je 50 Mol-% AgCl und AgBr mit einem mittleren Teilchendurchmesser von 0,86 µm erhalten. Die Emulsion wird geflockt, gewaschen und mit so viel Gelatine redispergiert, dass das Gelatine/AgN0₃-Gewichtsverhältnis 13 beträgt. Anschließend wird bei einem pH-Wert von 4,5 mit 3,4 µmol Goldchlorid pro mol Ag und 0,7 µmol Thiosulfat pro mol Ag bei 60 °C optimal gereift.

### II. Herstellung der leitfähigen Schichten:

Die fotografischen Schichtaufbauten werden auf einen Polyesterträger von 175 µm Dicke aufgetragen. Die Aufträge der Schichtbestandteile werden, sofern nicht anders vermerkt, in g/m² angegeben.

Im Falle der Silberhalogenidemulsion wird das AgNO₃ Äquivalent als Auftragsgröße angegeben. Beispiel 1 und Beispiel 2 unterscheiden sich nicht im Schichtaufbau, sondern nur in der gewählten nasschemischen Verarbeitung.

Schichtaufbau:
1. Schicht: 2,0 g Gelatine
2. Schicht: 2,0 g AgCI/Br, 550 µmol Sens-1 (bezogen auf mol Ag), 1,2 g Gelatine 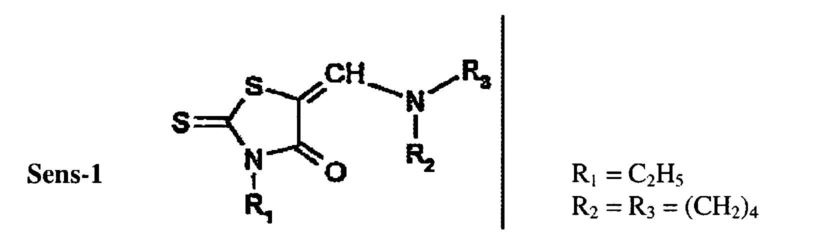
3. Schicht: 2,0 g Gelatine, 0,5 g Hydrochinon, 0,025 g Benzotriazol, 0,05 g Formalin

Durchführung der Versuche:

Auf das Material wird ein Linienraster mit 4 Linien/mm aufbelichtet, im den unten angegebenen Verarbeitungsprozessen verarbeitet und dann die Leitfähigkeit einer Leiterbahn von 4 Linien gemessen.

### Beispiel 1 (Vergleich):

| Entwickler | |
|---|---|
| Kaliumsulfitlösung, D = 1,45 | 375 ml |
| 1-Phenyl-4-methyl-3-pyrazolidinon | 0,8 g |
| Phenidon | 0,5 g |
| Hydrochinon | 30,0 g |
| Katiumcarbonat | 219,0 g |
| Ethylendiamintetraessigsäure, Na₄-Salz | 52,0 g |
| Kalilauge, D =1,50 | 15 ml |

Zum Gebrauch wird 1:7 mit Wasser verdünnt.

| Fixierbad | |
|---|---|
| Ammoniumthiosulfat | 130 g |
| Natriumdisulfit | 10 g |
| Natriumacetat | 9 g |
| Essigsäure, 80 gew.-%ig mit Wasser auffüllen auf 1 Liter, pH 5,4. | 5,6 ml |

Die Probe wird anschließend während 10 Minuten mit destilliertem Wasser bei 40 °C gespült um Restsalze zu entfernen.

### Beispiel 2 (Erfindung):

| Entwickler | |
|---|---|
| Diethylenglykol | 50 ml |
| Kaliumdisulfit | 30 g |
| 4-Methyl-4-hydroxymethyl-1-phenyl-3-pyrazolidon | 10 g |
| Kaliumhydrogencarbonat | 3 g |
| Hydroxyethandiphosphonsäure, 60 gew.-%ige wäßrige Lösung | 1 ml |
| Nitrilotriessigsäure | 15 g |
| Kaliumcarbonat | 250 g |
| Natriumisoascorbat | 150 g |
| Kaliumbromid | 15 g |
| Benzotriazol mit Wasser auffüllen auf 1 Liter , pH 10,75 | 0,2 g |

Zum Gebrauch wird 1:5 mit Wasser verdünnt. Nach dem Verdünnen beträgt der pH-Wert 10,5.

| Fixierbad | |
|---|---|
| Ammoniumthiosulfat | 130 g |
| Natriumdisulfit | 10g |
| Natriumacetat | 9 g |
| Essigsäure, 80 gew.-%ig mit Wasser auffüllen auf 1 Liter, pH-Wert 5,4. | 5,6 ml |

Die Probe wird anschließend während 10 Minuten mit destilliertem Wasser bei 40 °C gespült um Restsalze zu entfernen.

Zum Vergleich wird eine reine Gelatineschicht mit selbem Auftrag bezogen auf Gelatine gemessen, die Probenlänge beträgt 1 cm, die Messwerte sind in S/cm angegeben. Zum Vergleich: Silber: 6,2 * 10⁵ S/cm

| Probe | Messwert in S/cm |
|---|---|
| Gelatine | 6 * 10⁻¹² |
| Beispiel 1 (Vergleich) | 3 * 10⁻⁸ |
| Beispiel 2 (Erfindung) | 7 * 10⁻² |

Das in der Figur 4 gezeigte Verbundmaterial 301 weist eine mittlere Schicht 302, eine obere Schicht 303 und eine untere Schicht 304 auf. Das Verbundmaterial 301 ist mittels seiner unteren Schicht 304 auf ein Trägermaterial 305 appliziert. Im vorderen Bereich 306 löst sich die untere Schicht 304 bereits von dem Trägermaterial 305 ab. In der mittleren Schicht 302 sind in diesem Ausführungsbeispiel Carbon-Fullerene 340 eingebunden.

Im folgenden werden beispielhaft vorteilhafte Anwendungsbereiche des vorliegenden Verbundmaterials 301 erläutert.

Beispiel 1: Formulierung und Herstellung von NCF-Compounds für optische Reflexions- und Diffusions-Schichten zur räumlichen Bilddarstellung.

Ein optisches Acrylat-Gießharzsystem für den Einsatz in speziellen Verbundscheiben zur Wärme- und UV-Dämmung in Fassadenbereichen, Trenn- und Darstellungsflächen u.a. (Methylmetacrylat, n-Butylacrylat, 2-Ethylhexylacrylat, 2-Ethylhexylmetacrylat), Siedebereich > 100,0 °C, Dampfdruck ca. 47,o - 53,0 hPa) wurde mit einem Nano-Compound (Nano-Diamand Modifikation 2 - 0,025/1,0 Gew.% und einem hydrophoben Fest-Aerosil/R972 - 1,0/3,0 Gew.% sowie transparenzverbesserndes Blaupigment - 0,01/0,1 Gew.%) unter hochenergetischem Ultraschall homogen dispergiert (1,7 - 3,5 Stunden, < 50 °C) und stabilisiert.

Vorzugsweise wird das Acrylatgießharzsystem mit einem Kaskadengießer auf Triacetat- oder Polycarbonatfolie gegossen, wobei aus Haftungsgründen eine Gelatineschicht mit aufgebracht wird.

Im nachfolgenden sind zwei Schichtaufbaubeispiele beschrieben.

**Schichtaufbau 1:**

| | Schichtdicke nass |
|---|---|
| Gelatine (10%) | 10 µm |
| Gießlösung 1 | 20 µm |
| Trockenschichtdicke: 13,8 µm | |

**Schichtaufbau 2:**

| | Schichtdicke nass |
|---|---|
| Gelatine (10%) | 10 µm |
| Gießlösung 1 | 10 µm |
| Trockenschichtdicke: 6,4 µm | |

Zu besonders günstigen Eigenschaften kommt man durch vierschichtige Aufbauten.

**Schichtaufbau 3:**

| | Schichtdicke nass |
|---|---|
| Gelatine (10%) | 30 µm |
| Gießlösung 1 | 60 µm |
| Gießlösung 2 | 60 µm |
| Gießlösung 3 | 60 µm |
| Trockenschichtdicke: 158 µm | |

**Schichtaufbau 4:**

| | Schichtdicke nass |
|---|---|
| Gelatine (10%) | 30 µm |
| Gießlösung 1 | 60 µm |
| Gießlösung 2 | 60 µm |
| Gelatine (10%) | 30 µm |
| Trockenschichtdicke: 92,04 µm | |

Beispiel 2: Herstellung und Verwendung von Nano-Partikel kombinierten multi-funktionalen NCF-Compounds zur Verbesserung der mechanischen Eigenschaften von Lack-Schichten (Coatings) am Beispiel eines 2K-PUR-Matt-Lacksystems.

Die Modifizierung fertiger Lacksysteme mit NCF-Partikeln erfolgt auf indirektem Wege, indem man die Nano-Partikel zunächst in einem möglichst polaren und gering viskosen Lösungsmittel, welches bereits Bestandteil des Lackes ist, vordispergiert. Diese Vordispergate werden zur Modifizierung von Lack-Systemen eingesetzt.

Zur Modifizierung des 2K-PUR Mattlackes wird ein n-Butylacetat Vordispergat eingesetzt, in dem 10% monokristalline NCF-Partikel und 2% des Dispergierhilfsmittels Disperbyk-2150 (Lösung eines Block-Copolymers mit basischen pigmentaffinen Gruppen) enthalten sind. Die Dispergierung der monokristallinen Partikel erfolgt zunächst in einem Ultraschallbad (2 x 600 W/Per, 35 kHz) und anschließend mit einer Ultraschall-Durchflußapparatur (HF Ausgangsleistung 200 W, 20 kHz). Zur Entfernung etwaiger Verunreinigungen wird ein Sieb mit einer Maschenweite von 65 µm eingesetzt.

500 g des 2K-PUR Lackes (Komponente 1) werden zunächst in einem Becherglas vorgelegt, und nacheinander mit 100 g Sub-µm-Glasflakes (Glasplättchen aus Borsilikatglas, mittlere Größe 15 µm) und 15 g nanopartikuläres Aerosil^{®} R972 (hydrophobiertes, pyrogenes SiO₂, mittlere Größe der Primärteilchen 16 nm) versetzt. Die Dispergierung der Additive erfolgt im Ultraschallbad - hier: Glasplättchen 30 min und Aerosil^{®} R 972 60 min. Anschließend werden 5 g des n-Butylacetat Vordispergates eingerührt, und erneut im Ultraschallbad 60 min homogenisiert. Das fertiggestellte Nano-Compound führt zu entsprechenden multifunktionalen Verbesserungen der komplexen mechanischen Kenn- und Leistungsdaten des Matt-Lacksystems.

Die Applikation (Enabling) des modifizierten Lackes erfolgt entsprechend den Hersteller-Angaben, indem man die modifizierte Komponente 1 mit der vorgeschriebenen Menge Härter (Komponente 2) versetzt.

Besonders günstige Eigenschaften erzielt man, wenn die monokristallinen NCF-Partikel und 2% des Dispergierhilfsmittels Disperbyk-2150 (Lösung eines Block-Copolymers mit basischen pigmentaffinen Gruppen) mittels eines Kaskadengießers auf eine Unterlage aufgebracht wird und diese Folie als Schutz auf den Lack aufgebracht wird.

Getestet wurden unter anderem vergleichende Veränderungen der Oberflächentexturen (Mattlacke haben eine so unregelmäßige Feinstruktur der Oberfläche, dass das Licht in alle Richtungen gestreut wird, und kaum noch Spiegelwirkung vorhanden ist), der komplexen mechanischen Charakteristika sowie die Vernetzungsdichte des modifizierten und des Referenz-Lacksystems (nicht modifiziert).

Die Bewertung der Oberflächentextur nach Behandlung mit Stahlwollflies (maschinell), mit handelsüblicher Schleif- und Polierpaste erfolgte mikroskopisch bei 100facher Vergrößerung, und über die Bestimmung der Rauhigkeitswerte mittels Perthometer M4Pi der Firma Mahr nach DIN EN ISO 4287.

Die ermittelten Rauhigkeitswerte - insbesondere die Mittenrauhwerte Rₐ - verweisen auf eine wesentliche Verbesserung der Abriebfestigkeit sowie der Martens-Härte des modifizierten Lackes. Die Textur (Mattigkeit) der Lackoberfläche unterliegt im Vergleich zum Referenz-Lack nach den mechanischen Belastungen keine bzw. nur unwesentliche Veränderungen. Diese Ergebnisse werden durch die mikroskopischen Auswertungen wiedergegeben.

Erzielt wurden Verbesserungen der Abriebfestigkeit sowie der Oberflächen-Textur der NCF-modifizierten Lack-Systeme, die Erhöhung der Martens-Härtewerte und die Verbesserung der Scheuerbeständigkeit.

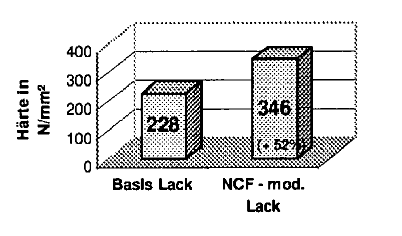

Martenshärten im Vergleich:

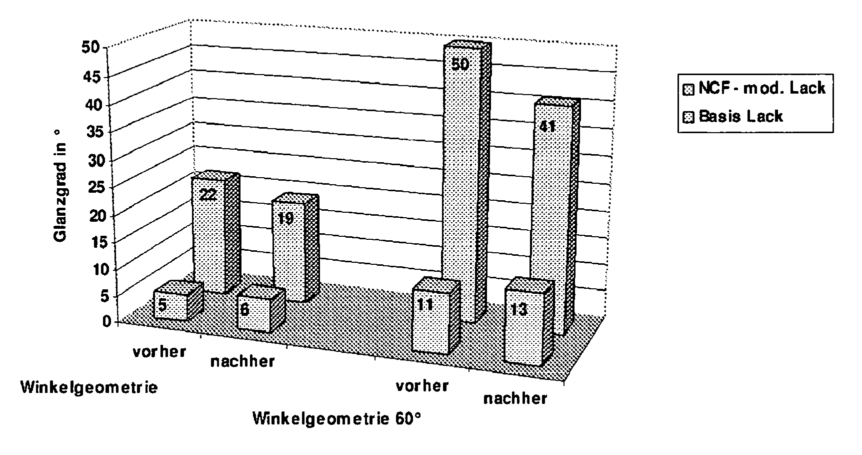

Scheuerbeständigkeiten im Vergleich:

Beispiel 3: Herstellung und Verwendung von Nano-Partikel kombinierten multi-funktionalen NCF-Compounds zur Verbesserung der tribologischen Eigenschaften (Gleiteigenschaften) von Gleitlacksystemen (Trocken-Schmiermittel) an Beispiel eines NCF-modifizierten Acrylat-Lackes auf Wasserbasis.

Die Modifizierung fertiger Lacksysteme mit NCF-Partikeln auf indirektem Wege, indem man die Nano-Partikel zunächst in einem möglichst polaren und gering viskosen Lösungsmittel, welches bereits Bestandteil des Lackes ist, vordispergiert. Diese Vordispergate werden zur Modifizierung von Lack-Systemen im weiteren eingesetzt.

Der Acrylat-Lack setzt sich aus zwei Komponenten zusammen. Komponente 1 enthält unter anderem die Acrylkomponente (Mowilith), welche sehr scherempfindlich ist. Aus diesem Grunde wird hier die zweite Komponente modifiziert, deren Bestandteile im Wesentlichen zur Viskositätseinstellung (Verdicker) fungieren.

Mischungsverhältnis: Komponente 1 - 86,4 Teile; Komponente 2 - 13,6 Teile.

Zur Modifizierung der Komponente 2 wird ein wässriges Vordispergat eingesetzt, in dem 5% monokristalline NCF-Partikel enthalten sind. Die Dispergierung der monokristallinen Partikel erfolgt zunächst in einem Ultraschallbad (2 x 600 W/Per., 35 kHz) und anschließend mit einer Ultraschall-Durchflußapparatur (HF Ausgangsleistung 200 W, 20 kHz). Zur Entfernung etwaiger Verunreinigungen wird ein Sieb mit einer Maschenweite von 38 µm eingesetzt.

15,3 g der Komponente 2 werden mit 200 g des wässrigen Vordispergates versetzt, und zur Viskositätseinstellung 75% des Wassers durch Temperierung auf 100 °C entfernt.

Die modifizierte Komponente 2 wird anschließend zu 85 g der Komponente 1 eingerührt. Zur Homogenisierung und Stabilisierung wird der modifizierte Lack 30 min im Ultraschallbad behandelt, mit 1,8 g Tamol^{®} NN 8906 (Naphthalinsulfonsäure-Kondensationsprodukt) versetzt und erneut 30 min im Ultraschallbad dispergiert.

Etwaige Verunreinigungen werden mit einem Sieb der Maschenweite von 180 µm entfernt. Der fertig modifizierte Lack enthält 6,5 Gew-% NCF-Partikel und 1,3 Gew-% Tamol^{®} NN 8906. Vorzugsweise wird der Acrylatlack mit einem Kaskadengießer auf Triacetat- oder Polycarbonatfolie gegossen, wobei aus Haftungsgründen eine Gelatineschicht mit aufgebracht wird.

Im nachfolgenden sind zwei Schichtaufbaubeispiele beschrieben:

**Schichtaufbau 1:**

| | Schichtdicke nass |
|---|---|
| Gelatine (10%) | 10 µm |
| Gießlösung 1 | 20 µm |
| Trockenschichtdicke: 13,8 µm | |

**Schichtaufbau 2:**

| | Schichtdicke nass |
|---|---|
| Gelatine (10%) | 10 µm |
| Gießlösung 1 | 10 µm |
| Trockenschichtdicke: 6,4 µm | |

Durch die Modifizierung verbessern sich die Gleitreibungswerte im Vergleich zum nicht modifizierten Lack um mehr als das doppelte, wobei die guten Abriebsfestigkeiten (Taber-Abraser-Test) des Acrylat-Lackes beibehalten werden. Im Vergleich zu kommerziellen PTFE- und MoS₂-Gleitlack Systemen weist der NCF-modifizierte Acrylat-Lack bzgl. der Gleitreibungswerte eine geringe Besserstellung auf, wobei sich hier die Abriebfestigkeit im Mittel um den Faktor 6 erhöht.

Ein Produktvorteil, der sich für den Anwender beim Einsatz von Gleitlacken zur Trockenschmierung vor allem in einer erhöhten Langzeit-und Lebensdauer-Schmierung und wirtschaftlichen Wertsteigerung auswirkt.

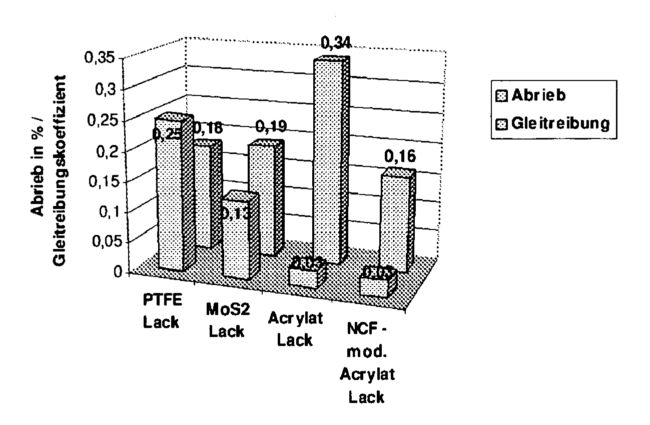

Beispiel 4: Herstellung und Verwendung einer wässrigen Nano-Suspension (Nano-Compound) für das Ultra-Präzisions-Polishing auf der Basis von Poly-NCF am Beispiel des Korngrößen-Bereiches 0- 0,5 µm für hochtechnologische Anwendungen mittels Träger-Pad (Basis-Material: Wasserbasiertes Polyacrylat).

Zur Herstellung dieser Nano-Suspension wird als Vorstufe eine ca. 2%ige, pH-neutrale Basis-Suspension eingesetzt, welche für den speziellen Anwendungsfall auf ca. 1,5% verdünnt und mit verdünnter Natronlauge auf pH 8 eingestellt wird. Die Basis-Suspension setzt sich aus dem Poly-NCF-System (0-0,5 µm), destilliertem Wasser und den Stabilisatoren und Konsistenzregler Polyvinylpyrrolidon (PVP bzw. Polyvidon 25 (LAB), und nano-partikulärem Aerosil^{®} A300 (pyrogenes SiO₂, mittlere Größe der Primärteilchen 7 nm) zusammen.

Zur Herstellung der Suspension werden erfindungsgemäß 100 g der Poly-NCF-Partikel portionsweise in 5 kg Wasser eingerührt und zunächst 3 h in einem Ultraschallbad (2 x 600 W/Per., 35 kHz) dispergiert. Zur weiteren Dispergierung wird das Dispergat anschließend 45 min mit einer Ultraschall-Durchflußapparatur (HF Ausgangsleistung 1000 W, 40 kHz) behandelt. Die Abtrennung etwaiger Verunreinigungen erfolgt mit einem Sieb der Maschenweite von 38 µm.

Die Stabilisierung erfolgt durch Zugabe von 250 g Aerosil^{®} A300 und 10 g einer 5%igen, wässrigen PVP-Lösung. Anschließend wird der Ansatz erneut 45 min mit der Ultraschall-Durchflußapparatur dispergiert.

Die Herstellung der speziellen pH 8 Suspension - Ansatz ca. 4,8 kg - erfolgt durch Verdünnung von 3,6 kg der Basis-Suspension mit 1,2 kg destilliertem Wasser (im Verhältnis 3:1, w:w) und anschließender Homogenisierung im Ultraschallbad für 15 min. Der pH-Wert der Suspension wird mit einer 1,5%igen Natronlauge auf pH 8 ± 0,2 eingestellt. Richtwert ca. 9 ± 2 ml verdünnte Natronlauge /kg Suspension.

Zusammensetzung des Nano-Compound in Gewichtsprozent:

| | |
|---|---|
| Poly-NCF (0-0,5 µm): | 1,4% |
| dest. Wasser: | 95,0% |
| Aerosil^{®} A300 : | 3,6% |
| Polyvidon: | 0,007% |
| NaOH (s) : | 0,012 ± 0,004% |

Beispiel 1 (Vergleich):

Vorzugsweise wird die Nano-Suspension mit einem Kaskadengießer auf Triacetat- oder Polycarbonatfolie gegossen, wobei aus Haftungsgründen eine Gelatineschicht mit aufgebracht wird.

Im nachfolgenden sind zwei Schichtaufbaubeispiele beschrieben.

**Schichtaufbau 1:**

| | Schichtdicke nass |
|---|---|
| Gelatine (10%) | 10 µm |
| Gießlösung 1 | 20 µm |
| Trockenschichtdicke: 13,8 µm | |

**Schichtaufbau 2:**

| | Schichtdicke nass |
|---|---|
| Gelatine (10%) | 10 µm |
| Gießlösung 1 | 10 µm |
| Trockenschichtdicke: 6,4 µm | |

Erzielte Test-Parameter und Leistungs-Charakteristika: Einsatzgebiet: Ultrapräzise Endpolitur von planen Spezial-Stepper-Optiken aus CaF₂.

Testversuche:

Versuchsvorbereitung:

Ein rundes CaF₂ - Teil bei dem ein kleiner Bereich mit Schutzlack abgedeckt wird, um so den Zustand vor Ätz-/Löseangriff des Poliermittels zu konservieren.

Versuchsablauf:

Die zuvor angegebene Spezialoptik wird nach einem Standardverfahren zur Hälfte mit einem rotierendem Werkzeug, belegt mit einem weichen Poliertuch, bearbeitet, um Konstantabtrag zu erreichen. Bearbeitung erfolgt in senkrechten Mäanderbahnen vom linken Rand beginnend. Ca. ½ Liter der Suspension wird im Umlauf hinzu gegeben. Bearbeitungszeiten lagen zwischen 30 Minuten bis 5 Stunden. In gleichem beschriebenem Ablauf wurden Versuche mit Wettbewerbs-Standard-Produkten als Vergleichsbasis durchgeführt.

Ergebnisse:

Mittlerer Abtrag mit dieser neuen Suspension 800 nm (Vergleich mit Standard-D 0,25er Suspensionen 300 bis 500 nm)

Erreichte Mikrorauhigkeiten:
bei 2,5 x = 1,1 bis 1,2 nm (Vergleich mit Standard D 0,25 = 1,3 bis 1,7 nm)
bei 20 x = 0,6 bis 0,7 nm (Vergleich mit Standard D 0,25 = 1,1 bis 1,7 nm)

Kratzerstatus:

Bei diesen neuen Systemen (gemessen im Dunkelfeldmilcroskop - 200fache Vergrößerung) keine sichtbaren Kratzer (Vergleich mit Standard D 0,25 = deutliche und mehr erhebliche Kratzer sichtbar).

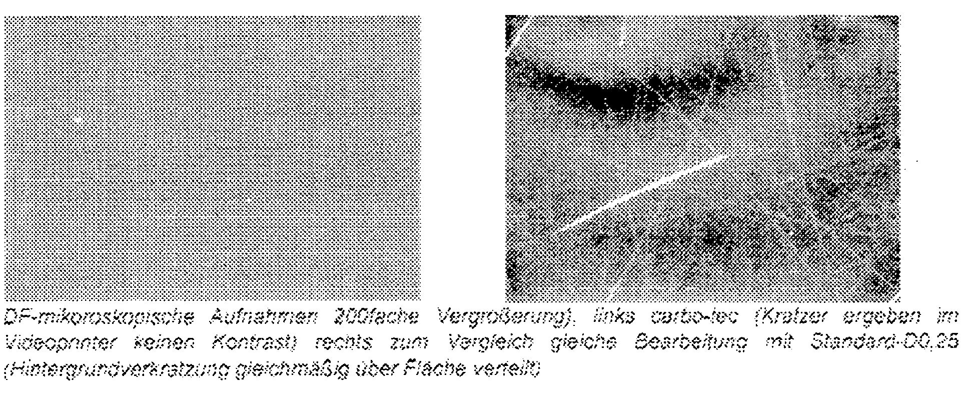

Beispiel 5: Nano-Compounds auf der Basis von NCF für das Heat-Management (Wärmeleit-Folien und -Schichten).

Die Verwendung neuer Generationen von Bauelementen der Leistungselektronik sowie die Realisierung innovativer Systemintegrationen bei wachsender Miniaturisierung und Steigerung der Prozeßgeschwindigkeiten, vorrangig in Bereichen der motiven Steuerung und Regelung, macht unter anderem den Einsatz von Wärmeleit-Systemen mit zum Teil wesentlich erhöhten Anforderungen an die Kenn- und Leistungscharakteristika erforderlich.

Nachstehende Tabelle 1 zeigt ausgewählte und gegenwärtig angestrebte Rahmenparameter.

Insbesondere die angezielte Erweiterung der thermo-konduktiven Bandbreite in Bereiche von über 5 W/mK (bis 20 W/mK) machen die Entwicklung innovativer wärmeleitender Filler-Systeme notwendig, die optimale Heat-sink- und vor allem Wärmeübergangseffekte an den entsprechenden Phasen- und Kontaktgrenzen bei erhöhten Operationstemperaturen des Gesamtkomplexes gewährleisten.

Die erforderliche Voraussetzung dafür ist: Die Formulierung speziell strukturierter und kombinierter Werkstoffverbunde sowie ihre gezielte Einbringung (Enabling) in das jeweilige Gesamtsystem der Träger-Matrix.

Tabelle 1: Erwünschte Materialeigenschaften

**Wärmeleitender hochflexibler Kleber**

| **Eigenschaft** | **Stand der Technik** | **Erwünscht** |
|---|---|---|
| Anwendung | Kühlkörper | Heat sink attach |
| Substrat | Al,Cu | Al, Cu |
| Komponenten | LTCC (Low Temperature Cofired Ceramics) | LTCC, DBC (Low Temperature Cofired Ceramics, |
| Geometrie | bis zu 2 inch | bis zu 2 inch |
| Kopplungsmethode | n.a. | n.a. |
| Zahl der Kopplungen | n.a | n.a. |
| Verarbeitung | Pin Transfer | Pin Transfer |
| Viskosität bei Raumtemperatur | | < 100Pas @ D = 1 s⁻¹, nicht thixotrop |
| Angestrebte Topfzeit | | > 3 Tage |
| Gewünschte Aushärtungszeit | | < 30 min bei 150 °C |
| Dielektrische Durchschlagsfestigkeit | > 20 kV/mm | > 20 kV/mm |
| Elektrische Leitfähigkeit | k.A. | k.A. |
| Wärmeleitfähigkeit | (2-3)W/mK | (5 - 20) W/mK. |
| Laststrom | k.A | k.A. |
| CTE | | |
| Glasübergangstemperatur | < -45 °C | < -45 °C |
| Modulus | | |
| Bruchdchnung | 30 % | > 70 % |
| Adhäsionskraft | > 2 N/mm² | > 2 N/mm² |
| Arbeitstemperatur | bis 150°C | >165 °C |
| Topfzeit | | |
| Umgebungsbedingungen (Klima) | -40°C/+ 150°C | -40°C/+ 165°C |
| Mechanische Bedingungen | | 40 g (Vibration) |

Als Matrix-Systeme werden zwei beispielhaft ausgewählte Formulierungen, auf der Basis cycloaliphatisches Epoxid (System a) sowie von Polymer-Silikonen (System b) betrachtet (Abstract 1), die unterschiedliche technologische Realisierungsansätze erforderlich machen.

Vorzugsweise wird die NCF-Lösung mit einem Kaskadengießer auf Triacetat- oder Polycarbonatfolie gegossen, wobei aus Haftungsgründen eine Gelatineschicht mit aufgebracht wird.

Im nachfolgenden sind zwei Schichtaufbaubeispiele beschrieben.

**Schichtaufbau 1:**

| | Schichtdicke nass |
|---|---|
| Gelatine (10%) | 10µm |
| Gießlösung 1 | 20 µm |
| Trockenschichtdicke: 13,8 µm | |

**Schichtaufbau 2:**

| | Schichtdicke nass |
|---|---|
| Gelatine (10%) | 10 µm |
| Gießlösung 1 | 10 µm |
| Trockenschichtdicke: 6,4 µm | |

Auszug 1: Anforderungen an thermisch leitende Filler aus Sicht der Formulierung.

Das fertige System sollte hochflexibel sein. Daher darf der Filler die Steifigkeit des Systems nicht erhöhen. Der ionische Anteil der fertigen Formulierung sollte so klein wie möglich sein. Verschiedene Basispolymere müssen getestet werden. Daher müssen die Fillermischungen mit den folgenden organischen Verbindungen kompatibel sein.

System a: Dieses System basiert auf einem Cycloaliphatischen Epoxid. Die chemische Formel ist nachstehend gezeigt. Dieses Harz hat eine Viskosität von 300 mPa s und eine Dichte von 1,16 g/cm³. Unter dem Einfluss von Säuren oder Basen beginnt es zu polymerisieren.

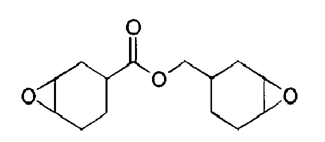

Die Fillermischung kann ein trockenes Pulver oder eine Mischung des Fillers in diesem Harz sein. Bis zu 80 Gewichts-% anderer organischer Verbindungen werden dem System zugefügt. Der Anteil des Fillers sollte in dieser Mischung so hoch wie möglich sein, sonst besteht die Gefahr, dass die Mischung nicht im erforderlichen Bereich variiert werden kann.

System b: Verschiedene Polymere auf Silikonbasis werden als flexibles Basispolymer verwendet. Da die Eigenschaften und Aushärtebedingungen von Silikonen nur über unterschiedliche Molekulargewichte und Art/Zahl der funktionellen Gruppen verändert werden können, ist es vorteilhaft, wenn der Filler ein trockenes Pulver ist. Dieses Pulver ist vorzugsweise mit Silikon-Öl (poly(dimethylsiloxane), PDMS) kompatibel.

Flüssige Mischungen sind eher unvorteilhaft, meistens nicht möglich, da dies für jedes der zahlreichen, zu testenden reaktiven Silikone eine separate Mischung bedeuten würde. Es wird vorliegend ein Verfahren benötigt, mit dem der trockene Filler in der Silikonmischung verteilt werden kann.

Formulierung optimierter Wärmeleit-Compounds (Varianten):

Mehrkomponentiger Einsatz von morphologisch unterschiedlichen Wärmeleit-Fillern im Verbund mit hochstrukturierten Nano-Mono- und Nano-Polysystemen (NCF) zur gestaffelten Formierung der angestrebten optimalen thermokonduktiven Charakteristika sowie zur Gewährleistung der erforderlichen Wirtschaftlichkeit bei hohen Füllgraden.

Realisierung optimierter Kombinationen von Filler-Partilcelgrößen in breitbandiger Größenverteilung von 10/50 nm bis 15/23 µm mit mehrfach sich überschneidenden Verteilungsmaxima (Peaks). Aufbau von Compound-Formulierungen in Kombination von sphärischen, dendritischen, faserförmigen bzw. lamellaren Fillerpartikel- und Clusterformen. Ansteuerung (Dotierung) der erforderlichen Oberflächenaktivitäten vorrangig der im Compound eingesetzten hochstrukturierten Nano-Carbon-Fullerene.

Anpassung der Enabling-Technologie für die Compoundierung der Trägermatrix:

Compound-Auslegung für System a: Stabile monomerische Dispersion.

Compound-Auslegung für System b: Vorformuliertes Trockendispergat in homogener Form.

## Patentansprüche

1. Verbundmaterial aus mindestens drei Schichten, ***dadurch gekennzeichnet, dass*** wenigstens eine der Schichten Wirkstoffe, keramische Nanopartikel, Silbersalze oder nanopartikuläre Kohlenstoffmodifikationen aufweist.

2. Verbundmaterial nach Anspruch 1, ***dadurch gekennzeichnet, dass*** wenigstens eine der Schichten (2, 3, 4) medizinische Wirkstoffe (7) oder Bitterstoffe aufweist.

3. Verbundmaterial nach Anspruch 1, ***dadurch gekennzeichnet, dass*** wenigstens eine der Schichten (2, 3, 4) sensibilisierte Silbersalze (230) aufweist.

4. Verbundmaterial nach Anspruch 1, ***dadurch gekennzeichnet, dass*** wenigstens eine der Schichten (2, 3, 4) Carbon-Fullerene (340) aufweist.

5. Verbundmaterial nach einem der Ansprüche 1 bis 4, ***dadurch gekennzeichnet, dass*** wenigstens eine der Schichten (2, 3, 4) des Verbundmaterials (1) eine Schichtdicke mit einer Toleranz von weniger als +/- 10 %, vorzugsweise mit einer Toleranz von +/- 1 %, aufweist.

6. Verbundmaterial nach einem der Ansprüche 1 bis 5, ***dadurch gekennzeichnet, dass*** die Schicht (2), welche wenigstens einen Bestandteil, nämlich Wirkstoffe, insbesondere medizinische Wirkstoffe (7) oder Bitterstoff, keramische Nanopartikel (120), Silbersalze, insbesondere sensibilisierte Silbersalze (230), oder nanopartikuläre Kohlenstoffmodifikationen, insbesondere Carbon-Fullerene (340), aufweist, von wenigstens zwei weiteren Schichten (3, 4) umgeben ist.

7. Verbundmaterial nach einem der Ansprüche 1 bis 6, ***dadurch gekennzeichnet, dass*** wenigstens eine der Schichten (2) erste Bestandteile (7) aufweist, welche von weiteren Bestandteilen (8) weiterer Schichten (3, 4) verschieden sind.

8. Verbundmaterial nach einem der Ansprüche 1 bis 7, ***dadurch gekennzeichnet, dass*** wenigstens zwei der Schichten (2, 3, 4) voneinander verschiedene Schichtdicken aufweisen.

9. Verbundmaterial nach einem der Ansprüche 1 bis 8, ***dadurch gekennzeichnet, dass*** wenigstens eine der Schichten (2, 3, 4) eine keramische Suspension mit mehr als 30 Gewichts-%, vorzugsweise von mehr als 45 Gewichts-%, an keramischen Nanopartikeln (120) aufweist.

10. Verbundmaterial nach einem der Ansprüche 1 bis 9, ***dadurch gekennzeichnet, dass*** wenigstens eine der Schichten (2, 3, 4) Gelatine aufweist.

11. Verbundmaterial nach einem der Ansprüche 1 bis 10, ***dadurch gekennzeichnet, dass*** wenigstens eine der Schichten (2, 3, 4) einen Verdicker, ein Härtungsmittel für einen Bestandteil einer Schicht (2, 3, 4), wie etwa Gelatine, oder ein Vernetzungsmittel für einen Bestandteil einer Schicht (2, 3, 4), wie etwa Gelatine, aufweist.

12. Verbundmaterial nach einem der Ansprüche 1 bis 11, ***dadurch gekennzeichnet, dass*** wenigstens eine der Schichten (2, 3, 4) elektrisch leitfähig ist.

13. Verbundmaterial nach einem der Ansprüche 1 bis 12, ***gekennzeichnet, durch*** eine elektrisch isolierende Schicht (103, 104), welche als Isolator ein Polymer, wie etwa Gelatine, aufweist.

14. Verbundmaterial nach einem der Ansprüche 1 bis 13, ***dadurch gekennzeichnet, dass*** polymerhaltige Schichten ein wasserlösliches Polymer umfassen.

15. Verbundmaterial nach einem der Ansprüche 1 bis 14, ***dadurch gekennzeichnet, dass*** das Verbundmaterial (1) eine Folie, insbesondere eine keramische Folie, umfasst.

16. Verbundmaterial aus mindestens drei Schichten, ***dadurch gekennzeichnet, dass*** das Verbundmaterial mittels eines Kaskadengießers oder eines Vorhanggießers hergestellt ist.

17. Verbundmaterial nach Anspruch 16, ***dadurch gekennzeichnet, dass*** das Verbundmaterial (1) eines der Merkmale nach einem der Ansprüche 1 bis 16 aufweist.
